# EUROPEAN PATENT APPLICATION

(11) **EP 0 582 483 A2**
(43) Date of publication of application: **09.02.1994**
(21) Application number: 93306215.0
(22) Date of filing: 05.08.1993
(51) Int. Cl.: A61B 17/36, B08B 15/04

(54) **Smoke evacuators for use in surgery**

(30) Priority: 05.08.1992 ZA 925879; 15.06.1993 ZA 934237
(71) Applicant: Shaer, Winston Daniel, Cape Town, Cape Province (ZA)
(72) Inventor: Shaer, Winston Daniel, Cape Town, Cape Province (ZA)
(74) Representative: Laredo, Jack Joseph

(57) **Abstract**

Surgical procedures involving diathermy result in the creation of quantities of smoke. This smoke is noxious and can have live viruses in it. To prevent the smoke leaving the operative site and reaching the surgeon and other theatre staff a smoke evacuator is provided which includes a dome (28) of transparent material with a suction pipe (36) leading to the space below the dome. The suction pipe can be connected to the vacuum system of the operating theatre. The dome can form a magnifying glass and the inlet to the suction pipe can have the inlet end skew with respect to the axis of the pipe.

## Description

### FIELD OF THE INVENTION

THIS INVENTION relates to smoke evacuators for use in surgery.

### BACKGROUND TO THE INVENTION

During the use of surgical diathermy, a large volume of smoke is generated by electro-cautery of tissues and vessels. This smoke has not only been demonstrated to be mutagenic, but has also been shown to contain numerous organic compounds. Some of these are macroscopic particulate, eg pieces of fat and carbon, and others are not at this time identifiable by current analytical methods.

The smoke has also been demonstrated to contain the HIV and hepatitis virus.

During surgical procedures in which a considerable amount of electro-cautery is used, large volumes of smoke are generated and this, due to the noxious nature of the fumes, makes surgery extremely unpleasant for both the surgeon and operating room personnel.

It is thus obvious that electro-cautery smoke is a significant potential hazard, even if one that has only recently been recognized, to those that work in operating rooms.

Current methods of smoke evacuation are limited and extremely inefficient, and there is to the Applicant's knowledge only a simple instrument currently available. This instrument includes a metal pipe which protrudes from the diathermy pen and terminates above the diathermy blade. The pipe is open at one end, the end above the blade, and connected to a suction source at the other. Smoke is sucked into the open end of the pipe and removed. The device is inefficient and only a relatively small amount of smoke is cleared.

### BRIEF DESCRIPTION OF THE INVENTION

According to one aspect of the present invention there is provided a smoke evacuator which includes a smoke evacuating tube having an inlet end and an outlet end, the inlet end having an end surface which lies in a plane which is oblique with respect to the axis of the tube, and a carrier structure to which said tube is secured.

Said carrier structure can include a carrier rod which has a straight section, the rod turning down at the front end of said straight section and the lower end of the turned down section joining said tube.

The carrier structure can further include an elongate wand which has a socket at one end, the end of said straight section remote from the end at which the rod turns down fitting in said socket.

According to a further aspect of the present invention there is provided a smoke evacuator comprising walling defining a transparent dome, a smoke outlet leading through said walling, and a carrier structure for mounting the smoke evacuator on an operating instrument.

Said carrier structure can comprise a rod fixed to said dome, a vertical plate having a first hole in it through which said rod can be passed, an elongate plate extending at right angles to said vertical plate and including means for fastening it to an operating instrument, a second hole in said vertical plate for receiving the front end of said instrument, and a third hole in said vertical plate through which suction tubing can be passed for connection to said outlet.

Said outlet is preferably in the form of a short tube projecting horizontally from the outer face of said dome, the inlet to the tube being contiguous with the curved inner face of the dome whereby the inlet end of the smoke outlet is of larger cross sectional area than the remainder of the smoke outlet.

The first mentioned hole is preferably oversized with respect to the rod so that the rod can slide backwards and forwards in the hole and move pivotally in a vertical plane.

According to a still further aspect of the present invention there is provided a smoke evacuator for use during diathermy and other procedures that involve cutting by means of heat, the extractor comprising a cover of transparent material for location over the zone at which heating is taking place, a smoke outlet pipe, the pipe having a first portion which is below said cover and a second portion which extends away from said cover for connection to a suction source, and a plurality of apertures in said first portion of the pipe, said apertures forming the smoke inlets to the pipe.

Said pipe can also be open at the free end thereof to form another smoke inlet.

Said cover is preferably in the form of a downwardly open dome. In this form the pipe can be positioned adjacent the underside of the dome.

Said cover is preferably carried by a support which can itself be mounted on a diathermy or other instrument, the cover being displaceable rectilinearly with respect to the support thereby to enable its position with respect to said instrument to be adjusted, said cover further being mounted on the support so that it can tilt about an axis extending generally parallel to the direction in which the cover can move with respect to the support.

Where the cover is in the form of a dome, said support is preferably arcuate in shape, said dome including means defining an arcuate passage which receives said support. Said means can be a series of clips.

### BRIEF DESCRIPTION OF THE DRAWINGS

For a better understanding of the present invention, and to show how the same may be carried into effect, reference will now be made, by way of example, to the accompanying drawings in which:-
Figure 1 is a side elevation of a carrier forming part of a smoke evacuator;
Figure 2 is a top plan view of the carrier of Figure 1;
Figure 3 is an elevation taken in the direction of the arrow A in Figure 1;
Figure 4 is a vertical section through a smoke evacuator hood;
Figure 5 is a top plan view of the hood of Figure 4;
Figure 6 is a transverse section on the line VI-VI of Figure 4;
Figure 7 is a side elevation which diagrammatically illustrates the smoke evacuator fixed to a diathermy pen;
Figure 8 is a top plan view of the pen and evacuator;
Figures 9, 10 and 11 are, to a larger scale, a side elevation and opposite end views of a wand;
Figure 12 illustrates a structure for use at an operative site where space is limited;
Figure 13 is a side elevation of a diathermy instrument with a further form of smoke evacuator fitted;
Figure 14 is a side elevation of the diathermy instrument and the smoke evacuator of Figure 13;
Figure 15 is a top plan view of part of the smoke evacuator of Figures 13 and 14; and
Figure 16 is an underneath view of part of the smoke evacuator of Figures 13 and 14.

### DETAILED DESCRIPTION OF THE DRAWINGS

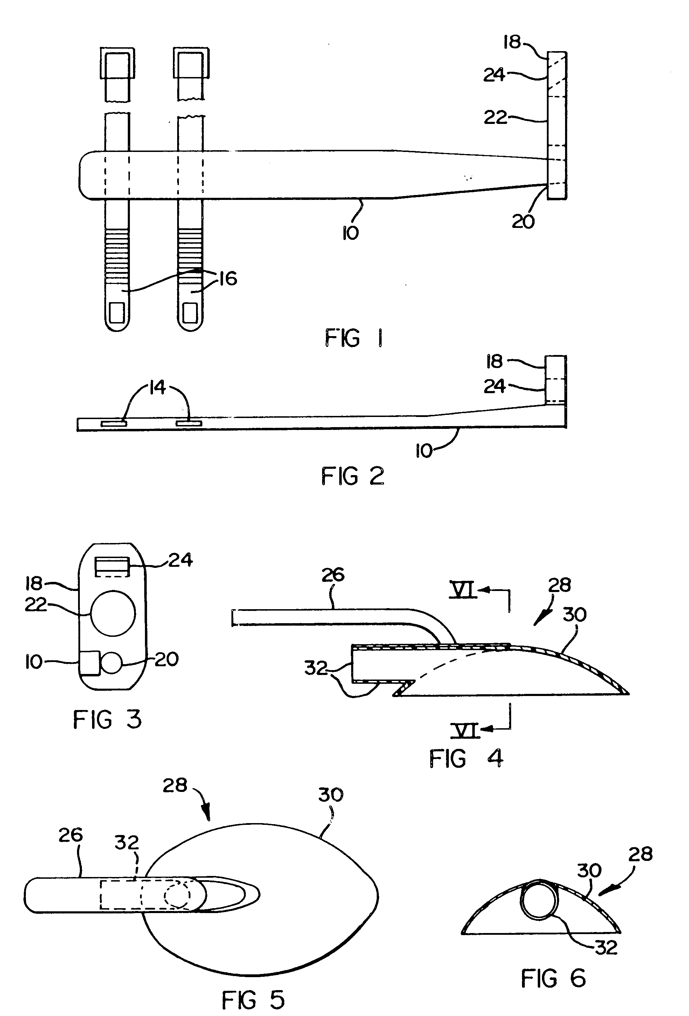

Referring firstly to Figures 1 to 3, the carrier illustrated is of elongate form and comprises a carrier plate 10. At its rear end the plate 10 is formed with two vertical passageways 14 (Figure 2). Straps 16 (shown in Figure 1) pass through the passageways 14 and, as will be described hereinafter, are used to connect the carrier to a diathermy pen.

The straps 16 are each of the type which has a head at one end with a passage passing through the head, and a toothed section at the other end. Within each passage there is at least one locking tooth. When the toothed section of a strap is pushed through the passage of that strap, its teeth interlock with the tooth in the passage and prevent the toothed section thereafter being withdrawn from the passage.

Whilst two straps 16 are shown, a single strap is adequate to secure the plate 10 firmly in position.

At the front end of the carrier plate 10 there is a mounting plate 18 which, as best seen in Figure 3, is offset with respect to the carrier plate 10. The mounting plate 18 has three holes 20, 22 and 24 therein. The lower hole 20 serves to receive the forward end of a diathermy pen, the centre hole 22 receives a suction tube, and the hole 24 receives the rod 26 (see Figures 4 etc) of a smoke evacuator hood which is generally designated 28. The holes 20 and 22 are both circular and their axes are at right angles to the faces of the plate 18. The hole 24, as shown in Figure 1, is angled so that its axis is at about 45° with respect to the faces of the mounting plate 18. It is also oversized with respect to the rod 26 so that, when the rod 26 is in position in the hole 24, it has a degree of freedom of movement in a vertical plane. The rod can, of course, also be moved backwards and forwards with respect to the plate 18. No lateral movement of the rod 26 with respect to the plate is permitted.

The hood 28 comprises the rod 26 and a dome 30 which is secured to the forward end of the rod 26 (see particularly Figure 4). It will be noted from this Figure that the rod curves downwardly at its front end to the point at which it is connected to the dome 30 and that the dome 30 is extended rearwardly by means of a short tube 32 which is integral therewith. The dome 30 and tube 32 are preferably injection moulded in transparent synthetic plastics material.

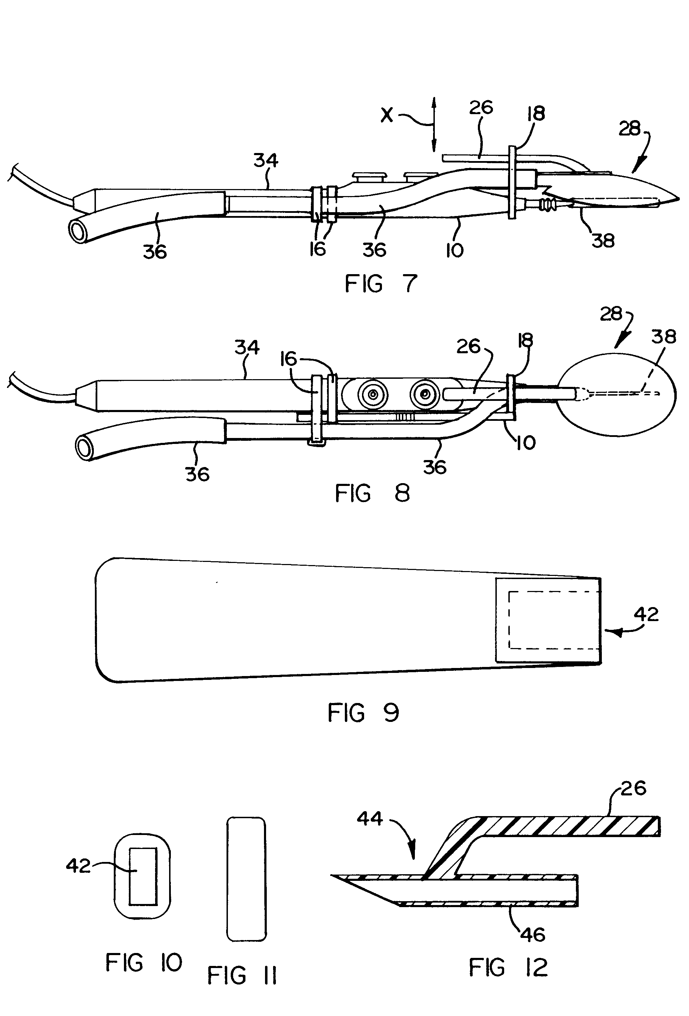

To use the smoke evacuator with a diathermy pen (shown at 34 in Figures 7 and 8) the leading end of the pen is pushed-through the hole 20 in the mounting plate 18. The rod 26 is in the hole 24 and tubing 36 (which is connected to the vacuum system of the operating theatre) is passed through the hole 22 and fitted onto the tube 32.

The right hand strap 16 is passed around the pen 34 and pulled tight. The left hand strap 16 is passed around the tubing 36 and the pen and also pulled tight.

It will be noted that the carrier plate 10 now lies alongside the pen 34 and that the cutter 38 of the pen 34 is underneath the hood 28. Because of the angled nature of the hole 24 the rod 26 can be manipulated, as shown by the double headed arrow X in Figure 7, to move the dome 30 closer to or away from the cutter 38.

Because the dome 30 is of transparent material, the operative site can readily be viewed through it. Smoke is drawn away via the tube 32 and tubing 36 and the dome prevents smoke, as well as solids and liquids that might be ejected from the operative site, from reaching the surgeon. The dome 30 can be designed to magnify the surgeon's view of the operative site.

In the event that the operative procedure does not involve the use of a diathermy pen, but it is still desired to use the smoke evacuator, then the wand of Figures 9, 10 and 11 is used in conjunction with the hood 28. The wand can be, for example, 200mm long and of the tapering configuration illustrated in Figure 9. At its narrower end there is a socket designated 42 which is dimensioned so that it can receive the end of the rod 26 which is remote from the dome 30.

For use at operative sites where there is little space eg in ear, nose and throat work, the structure 44 of Figure 12 can be used. The dome 30 is omitted and the rod 26 is connected to a tube 46. One end of the tube 46 is cut off at an oblique angle thereby providing an inlet to the tube the dimensions of which are greater than the cross sectional area of the tube. The suction tubing 36 is connected to the outlet end (the right hand end in Figure 12) of the tube 46.

The structure 44 can be mounted on the wand of Figures 9, 10 and 11 or on the carrier of Figures 1 to 3.

In a further form the hole 24 is omitted which means that the overall height of the plate 18 can be reduced. The rod 26 is also omitted. In this form two resilient arms extend forward from the plate 18, each arm having a ball or socket at the free end thereof. On the side faces of the tube 32 (Figures 4 to 6) or on the side faces of the tube 46 (Figure 12), there are two matching balls or sockets. The balls and sockets form joints which permit movement of the evacuator hood 28 or structure 44 with respect to the plate 18. The balls can be replaced by horizontal pivot pins if it is desired to restrict movement to a vertical plane. The resilience of the arms permits the evacuator hood 28 or structure 44 readily to be detached from the arms.

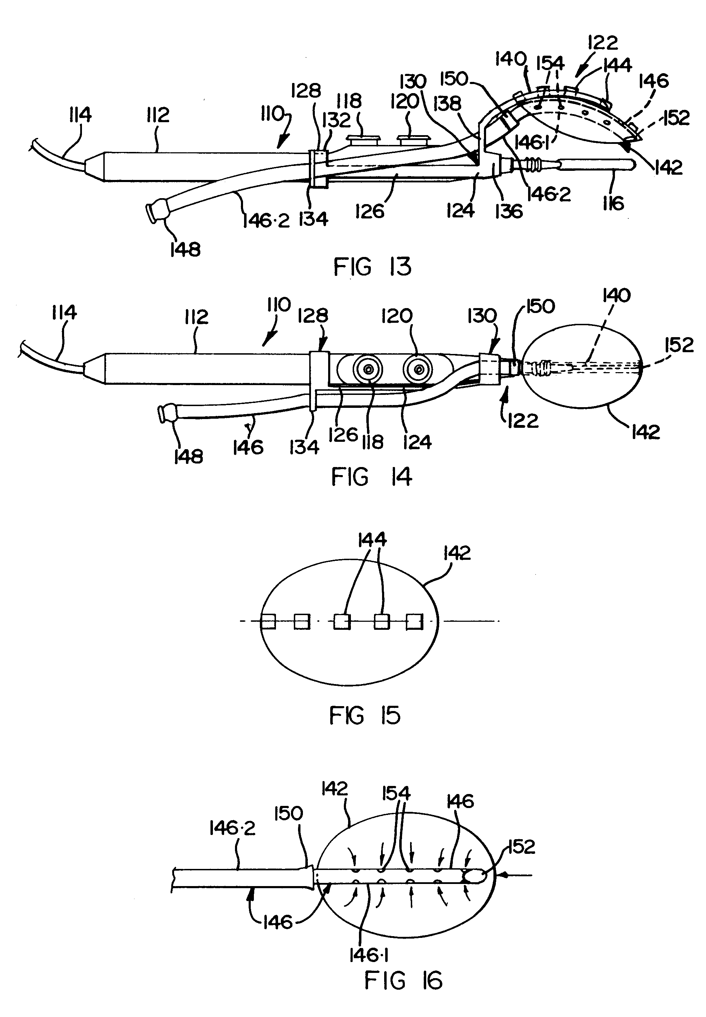

The diathermy pen illustrated in Figures 13-16 is the same as that illustrated in Figures 7 and 8, the pen being generally designated 110 and including an elongate body 112. A diathermy cable 114 emerges from one end of the body 112 and a diathermy blade 116 protrudes from the other end of the body 112. On the top of the body 112 are two buttons 118 and 120 which control operation of the pen 110.

Reference numeral 122 indicates a smoke evacuator which is releasably mounted on the body 112. The smoke evacuator 122 includes a carrier 124 which has an elongate portion 126 lying adjacent one of the side faces of the body 112, there being a rear mounting 128 at one end of the portion 126 and a front mounting 130 at the other end of the portion 126.

The rear mounting 128 is in the form of a clip 132 which can be forced downward onto the body 112. The clip 132 opens up as it is pressed onto the body 112, and then snaps closed onto the body. Integral with the clip 132 is a pipe guide 134. The front mounting 130 comprises two rings 136 and 138 one above the other, the lower ring 136 receiving the body 112 and the upper ring 138 being a pipe guide.

Above the upper ring 138 the carrier 124 is extended forwardly to constitute a flexible arcuate support 140 which overhangs the blade 116.

The smoke evacuator further includes a dome designated 142. The cover is of transparent synthetic plastics material, transparent polycarbonate or crystal styrene being the preferred materials. Any other suitable transparent synthetic plastics material can, however, be used.

The dome 142 is somewhat oval in shape in plan view being longer than it is wide. On the top face of the dome 142 is a series of C-shaped mounting clips 144 which receive the support 140. Fixed to the underside of the dome 142 is a smoke outlet pipe 146. The smoke outlet pipe 146 has a first portion which is below the dome 142 and a second portion which extends away from the dome for connection to a suction source (not shown). The pipe 146 passes through the rings 138 and 134. The connection between the smoke outlet pipe 146 and the pipe (not shown) which leads to the suction source is by way of a connecting nipple 148.

The pipe 146 is itself in two parts, the two parts being designated 146.1 and 146.2 and joining at a nipple designated 150.

The part 146.1 of the pipe 146 which is under the dome 142 is open at its free end to form a smoke inlet aperture 152. It furthermore has a plurality of apertures 154 in the wall thereof. At least the part 146.1 of this pipe 146 which is below the cover is of transparent material.

The carrier 124 is secured to the body 112 by pushing the front part of the body 112 through the ring 136. The clip 132 is then pressed downwardly onto the body 112 until it snaps closed about the body. The entire carrier 124 is then pushed away from the pen 116 so that the ring 136 is a tight fit on the tip of the body 112. The nipple 148 is then connected to the suction apparatus.

The smoke generated where the blade 116 is cutting is sucked into the pipe 146. It will be noted that the aperture 152 is close to the cutting point of the blade 116 and thus is well positioned to suck smoke away from the cutting point. The creation of a low pressure zone under the cover 142 causes smoke to be drawn upwardly into the space below the cover 142, then into the pipe 146 and be conveyed away.

Should a blood vessel accidentally be cut, the cover 142 intercepts, before it reaches the surgeon, any blood which tends to jet away from the operative site.

Because the support 140 is curved, and the clips 144 lie along a correspondingly curved line, the cover 142 can be moved towards or away from the diathermy blade 116. This affords the surgeon the opportunity of placing the cover 142 in the position with respect to the blade 116 which he judges will be most efficient from the point of view of extracting smoke, blood and carbon particles, and provides him with the best protection.

The flexibility of the support 140 enables the cover 142 to be rotated somewhat. It is thus possible to impart a degree of tilt to the cover 142 if the surgeon judges that this is what is required in a particular operative situation.

Whilst specifically intended for use with a diathermy pen, the smoke evacuator according to the present invention can be used in conjunction with other instruments which cut tissue using heat, whether generated in a metal blade or by means of a laser beam. In the case of a surgical laser, the body of the laser can pass through the cover so that part of the body lies below the cover. Alternatively the body can be entirely above the cover and the laser can focus through the cover.

It is also possible to fit a smoke evacuator according to the invention to diathermy forceps which are used for cauterizing a cut blood vessel, or to instruments used in endoscopic surgery.

The structure described not only efficiently and effectively removes electro-cautery smoke, but, in the forms which include the dome, also shields the user when the cautery knife is used in dissection and high pressure arterial vessels are divided. Not only the blood jet is blocked by the transparent dome but also any particulate matter that is generated in the procedure of electro-cautery. Furthermore this form increases the efficiency of smoke evacuation, by facilitating its collection and subsequent evacuation. The dome, being transparent, does not cause any obstruction to clear visualisation of the operative field. If magnification is built into the dome, this enhances visualisation and adds significantly to the advantageous features of the smoke evacuator.

In the drawings the pen 34, 110 has been shown with its control buttons uppermost and the carrier structure (10, 124/126) lying adjacent one of its side faces. The pen can if desired be rotated through 90 degrees so that the buttons are on one side and the carrier structure against what was previously the bottom face of the pen and has now become the side face. The blade must in these circumstances also be turned through 90 degrees.

## Claims

1. A smoke evacuator comprising walling defining a transparent dome, a smoke outlet leading through said walling, and a carrier structure for mounting the smoke evacuator on an operating instrument.

2. A smoke evacuator as claimed in claim 1, wherein said carrier structure comprises a rod fixed to said dome, a vertical plate having a first hole in it through which said rod can be passed, an elongate plate extending at right angles to said vertical plate and including means for fastening it to an operating instrument, a second hole in said vertical plate for receiving the front end of said instrument, and a third hole in said vertical plate through which suction tubing can be passed for connection to said outlet.

3. A smoke evacuator as claimed in claim 2, wherein said outlet is in the form of a tube projecting horizontally from the outer face of said dome, the inlet to the tube being contiguous with the curved inner face of the dome whereby the inlet end of the smoke outlet is of larger cross sectional area than the remainder of the smoke outlet.

4. A smoke evacuator as claimed in claim 3, wherein said first hole is oversized with respect to the rod so that the rod can slide backwards and forwards in the hole and move pivotally in a vertical plane.

5. A smoke evacuator which includes a smoke evacuating tube having an inlet end and an outlet end, the inlet end having an end surface which lies in a plane which is oblique with respect to the axis of the tube, and a carrier structure to which said tube is secured.

6. A smoke evacuator as claimed in claim 5, wherein said carrier structure includes a carrier rod which has a straight section, the rod turning down at the front end of said straight section and the lower end of the turned down section joining said tube.

7. A smoke evacuator as claimed in claim 6, wherein said carrier structure further includes an elongate wand which has a socket at one end, the end of said straight section remote from the end at which the rod turns down fitting in said socket.

8. A smoke evacuator for use during diathermy and other procedures that involve cutting by means of heat, the extractor comprising a cover of transparent material for location over the zone at which heating is taking place, a smoke outlet pipe, the pipe having a first portion which is below said cover and a second portion which extends away from said cover for connection to a suction source, and a plurality of apertures in said first portion of the pipe, said apertures forming the smoke inlets to the pipe.

9. A smoke evacuator as claimed in claim 8, wherein said cover is in the form of a downwardly open dome.

10. A smoke evacuator as claimed in claim 8, wherein said cover is carried by a support for mounting on a diathermy or other instrument, the cover being displaceable rectilinearly with respect to the support thereby to enable its position with respect to said instrument to be adjusted, said cover further being mounted on the support so that it can tilt about an axis extending generally parallel to the direction in which the cover can move with respect to the support.
